# EUROPEAN PATENT APPLICATION

(11) **EP 4 596 678 A1**
(43) Date of publication of application: **06.08.2025**
(21) Application number: 23870459.7
(22) Date of filing: 19.09.2023
(51) Int. Cl.: C12N 5/0775, A61K 35/28, C12N 5/10, C12N 5/078

(54) **ENGINEERED MESENCHYMAL STEM CELL AND USE THEREOF**

(30) Priority: 27.09.2022 CN 202211179684
(71) Applicant: Tsinghua University, Beijing 100084 (CN)
(72) Inventor: FU, Yangxin, Beijing 100084 (CN); LIANG, Yong, Beijing 100084 (CN)
(74) Representative: Boult Wade Tennant LLP
(86) International application number: PCT/CN2023/119557
(87) International publication number: WO 2024/067227

(57) **Abstract**

The present invention provides engineered mesenchymal stem cells (MSCs), a pharmaceutical composition containing the same, and use thereof in the manufacture of a medicament for treating cancer. The mesenchymal stem cells (MSCs) comprise an introduced NQO1 protein-coding sequence and/or an introduced hypoxia response element (HRE), and a nucleic acid encoding one or more immunostimulatory cytokines.

## Description

### TECHNICAL FIELD

The present invention relates to engineered genetically modified mesenchymal stem cells (MSCs) and use thereof in expressing immunostimulatory cytokines for tumor immunotherapy.

### BACKGROUND ART

Cancer is a disease posing a serious threat to human health, and abnormal proliferation or differentiation of cancer cells result in abnormal functionality, uncontrolled cell growth, local tissue invasion, and metastasis. Cancer cells express specific tumor neoantigens due to genetic mutations and can be recognized and killed by immune T lymphocytes, laying a basis for tumor immunotherapy. However, tumors have evolved various mechanisms, including upregulation of inhibitory immune checkpoint molecules and recruitment of immunosuppressive myeloid cells, so as to create an immunosuppressive microenvironment. After administration of an immunotherapy, lack of T lymphocyte growth factors (such as IL-2, IL-15) to maintain stimulation or reactivation can lead to T lymphocyte dysfunction and tumor recurrence. As an agonist for T lymphocytes and NK cells, IL-2 was the first cytokine approved by the FDA for the treatment of metastatic melanoma and renal cancer. However, the clinical application of IL-2 for cancer treatment has long faced challenges such as a short half-life and severe dose-dependent toxic side effects. More importantly, IL-2 can also expand immunosuppressive regulatory T cells (Tregs), limiting its anti-tumor efficacy. In order to address these issues, various strategies have been tried to construct IL-2 variants that selectively expand effector T cells but not Tregs. However, systemic administration of IL-2 variants may tend to expand T cells and NK cells in lymphoid tissue and non-tumor tissues, leading to severe toxicity. Therefore, it is important to deliver cytokines such as IL-2 or IL-2 variants specifically to the tumor microenvironment to effectively activate and expand lymphocytes while avoiding peripheral toxicity.

Within solid tumor tissue, stromal cells interact with tumor cells and immune cells to form a complex tumor microenvironment (TME). MSCs are an important source of tumor-associated stromal cells. In many cases, MSCs injected into animal bodies demonstrate a tumor homing ability. On one hand, MSCs can promote tumor progression, and suppress inflammation and immune responses. On the other hand, MSCs have a great potential to serve as a cellular carrier for delivering or producing anti-tumor molecules due to their pleiotropy in tumors. Certain recent studies have reported strategies for treating tumors with genetically modified MSCs. However, the application of MSCs in tumor therapy has encountered several serious challenges: first, how to transform immunosuppressive MSCs into anti-tumor MSCs; second, how to ensure that MSCs express anti-tumor factors specifically within the tumor to avoid toxic side effects caused by peripheral expression; and furthermore, the relatively short life of transplanted MSCs *in vivo* also affects their clinical use. Therefore, for clinical application, it is essential to enable genetically modified MSCs to extend their life *in vivo* and to controllably produce tumor therapeutic factors, thereby exerting the function of activating anti-tumor immune responses locally within the tumor.

### SUMMARY OF THE INVENTION

To address the aforementioned issues in the art, the inventors have precisely modified MSCs and obtained a genetically modified MSC with an enhanced tumor-treating ability, a stronger *in vivo* surviving ability, and capacity to express target gene products specifically within tumor tissues, and completed the present invention.

In the first aspect, the present invention provides an engineered mesenchymal stem cell (MSC) comprising in its genome an introduced NQO1 protein-coding sequence. In one embodiment, the introduced NQO1 protein-coding sequence enables the engineered MSC to overexpress the NQO1 protein. The overexpression of the NQO1 protein can extend the survival time of the engineered MSC within tumor tissues. In one embodiment, the NQO1 protein-coding sequence is introduced into the MSC's genome through lentiviral transduction, adenoviral transduction, or mRNA transfection to obtain the engineered MSC. In one embodiment, the genome of the engineered MSC further comprises an introduced regulatory element and an introduced target gene, wherein the expression of the target gene is regulated by the regulatory element.

In the second aspect, the present invention provides an engineered MSC comprising in its genome an introduced hypoxia response element (HRE), and optionally an introduced regulatory element and an introduced target gene located downstream of the HRE, wherein the expression of the target gene is regulated by the regulatory element. The HRE can activate the downstream regulatory element (e.g. a promoter) under a hypoxic condition within tumor tissues, thereby promoting the expression of the gene downstream of the regulatory element. In one embodiment, the HRE is introduced into the genome of the engineered MSC through lentiviral transduction, adenoviral transduction, or mRNA transfection. In one embodiment, the HRE together with the regulatory element (e.g. a promoter) and the target gene downstream of the HRE is introduced into the genome of the engineered MSC through lentiviral transduction, adenoviral transduction, or mRNA transfection. In one embodiment, in the genome of the engineered MSC, the HRE and the regulatory element and the target gene downstream of the HRE are operably linked. In one embodiment, the sequence of the HRE is derived from any of *Eno1, Epo,* VEGF-A, PGK, *Ldha,* ALDA, and GAPDH genes, preferably selected from SEQ ID NO: 2 and SEQ ID NOs: 6-11, and more preferably SEQ ID NO: 2 derived from the *Eno1* gene .

In the third aspect, the present invention provides an engineered MSC comprising in its genome: (i) an introduced NQO1 protein-coding sequence and (ii) an introduced hypoxia response element (HRE). In one embodiment, the genome of the engineered MSC further comprises an introduced regulatory element and an introduced target gene downstream of the HRE, wherein the expression of the target gene is regulated by the regulatory element. In one embodiment, in the genome of the engineered MSC, the HRE and the regulatory element and the target gene downstream of the HRE are operably linked.

In any of the above embodiments, when the engineered MSC further comprises a regulatory element and a target gene, the regulatory element may include a promoter.

In any of the above embodiments, when the engineered MSC further comprises a regulatory element and a target gene, the target gene may encode an anti-tumor protein, such as an immunostimulatory cytokine or an anti-tumor cytokine. In one embodiment, the target gene encodes any of IL-2, IL-1, IL-7, IL-21, IL-12, IL-15, and variants thereof. In one embodiment, the IL-1 is IL-1β. In one embodiment, the IL-2 variant is sumIL2.

The engineered MSC of the present invention can utilize MSC as a carrier to deliver immunostimulatory cytokines or anti-tumor cytokines specifically to the tumor microenvironment, thereby enhancing anti-tumor efficacy while avoiding peripheral toxic side effects caused by systemic administration of the cytokines. Moreover, no tumor-promoting activity of the engineered MSC of the present invention has been observed.

The fourth aspect of the present invention provides a pharmaceutical composition for treating cancer, comprising the engineered MSC of any of the first to third aspects, wherein the genome of the engineered MSC further comprises the introduced regulatory element and the introduced target gene, and the target gene encodes an immunostimulatory cytokine or an anti-tumor cytokine. In one embodiment, the pharmaceutical composition further comprises an immune checkpoint inhibitor and/or adoptive T cells that specifically recognize tumor cells.

The fifth aspect of the present invention provides a method for treating cancer, comprising administering a therapeutically effective amount of the engineered MSCs of any of the first to third aspects to a subject in need thereof, wherein the genome of the engineered MSC further comprises the introduced regulatory element and the introduced target gene, and the target gene encodes an immunostimulatory cytokine or an anti-tumor cytokine. In one embodiment, the subject is human. In one embodiment, the method further comprises administering an immune checkpoint inhibitor to the subject. In one embodiment, the method further comprises administering adoptive cell therapy (ACT) to the subject.

The sixth aspect of the present invention provides use of the engineered MSC of any of the first to third aspects in the manufacture of a medicament for treating cancer, wherein the genome of the engineered MSC further comprises the introduced regulatory element and the introduced target gene, and the target gene encodes an immunostimulatory cytokine or an anti-tumor cytokine. In one embodiment, the pharmaceutical composition further comprises a second anti-cancer agent, such as an immune checkpoint inhibitor and/or adoptive T cells that specifically recognize tumor cells.

In each of the embodiments of the fourth to sixth aspects, the regulatory element may include a promoter. In one embodiment, the promoter is a minimal SV40 promoter. In one embodiment, in the engineered MSC, the HRE, the regulatory element, and the target gene are linked operably. In one embodiment, the target gene encodes any of IL-2, IL-1, IL-7, IL-21, IL-12, IL-15, and variants thereof. In one embodiment, the IL-1 is IL-1β. In one embodiment, the IL-2 variant is sumIL2. In one embodiment, the cancer may be selected from B-cell lymphoma, bronchial cancer, prostate cancer, bladder cancer, brain or central nervous system cancer, peripheral nervous system cancer, esophageal cancer, cervical cancer, uterine or endometrial cancer, oral cancer, laryngeal cancer, salivary gland cancer, thymus cancer, adrenal cancer, osteosarcoma, chondrosarcoma, liposarcoma, testicular cancer, malignant fibrous histiocytoma, colorectal cancer, melanoma, gastric cancer, pancreatic cancer, lung cancer, liver cancer, renal cancer, cholangiocarcinoma, small intestine cancer or appendiceal cancer, squamous cell carcinoma, breast cancer, and ovarian cancer.

The present invention further provides use of the engineered mesenchymal stem cell of any of the first to third aspects as a carrier for delivering a target gene. In one embodiment, the carrier is used for delivering the target gene to tumor tissues. In one embodiment, the target gene is a tumor therapeutic gene, preferably a gene encoding an immunostimulatory protein or an anti-tumor protein, more preferably a gene encoding an immunostimulatory cytokine or an anti-tumor cytokine, such as the specific cytokines described above.

In other aspects of the present invention, provided are use of NQO1 protein for enhancing the survival of mesenchymal stem cells within tumor tissues; use of the hypoxia response element (HRE) for enhancing the expression of a target gene in mesenchymal stem cells under a hypoxic condition; and use of a combination of an NQO1 protein-coding sequence and a hypoxia response element (HRE) in the manufacture of a pharmaceutical composition which comprises mesenchymal stem cells carrying a target gene. In one embodiment, the hypoxic condition is the tumor microenvironment. In one embodiment, the genome of the mesenchymal stem cell comprises: (i) an introduced NQO1 protein-coding sequence, and/or (ii) an introduced hypoxia response element (HRE), as well as an introduced regulatory element and an introduced target gene downstream of the HRE, wherein the expression of the target gene is regulated by the regulatory element.

### BRIEF DESCRIPTION OF THE DRAWINGS

The specific embodiments of the present invention will be described with reference to the accompanying drawings, but the drawings and the specific embodiments hereinafter should not be interpreted as limiting the scope of the present invention. In the drawings:

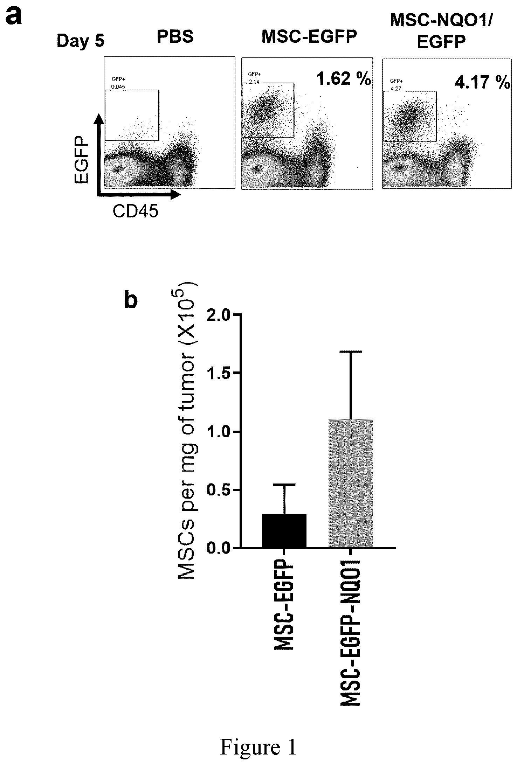

Figure 1 shows the survival of engineered MSCs expressing NQO1 within the tumor microenvironment: (a) a representative plot of the flow cytometry analysis result; (b) the number of surviving MSCs per unit weight of the tumor tissue.

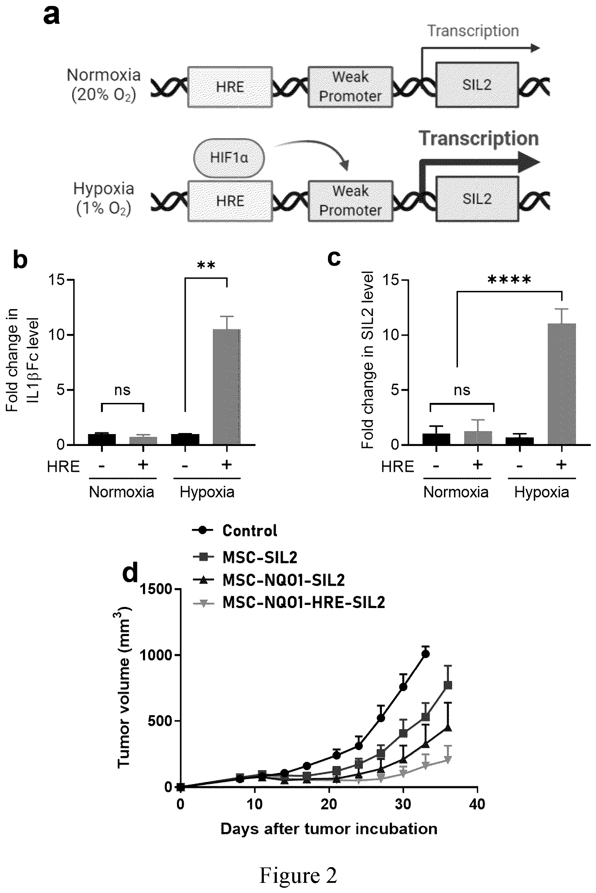

Figure 2 shows the effect of HRE-engineered MSCs on the expression of a target gene. (a) A schematic diagram of the working mechanism of HRE; (b) the expression of IL-1β in NQO1/HRE-engineered MSCs carrying an IL-1β gene under normoxic and hypoxic conditions; (c) the expression of sumIL-2 (SIL-2) in NQO1/HRE-engineered MSCs carrying a SIL-2 gene under normoxic and hypoxic conditions; (d) the anti-tumor effects of non-engineered MSCs, NQO1-engineered MSCs, and NQO1/HRE-engineered MSCs, all carrying a sumIL-2 (SIL-2) gene.

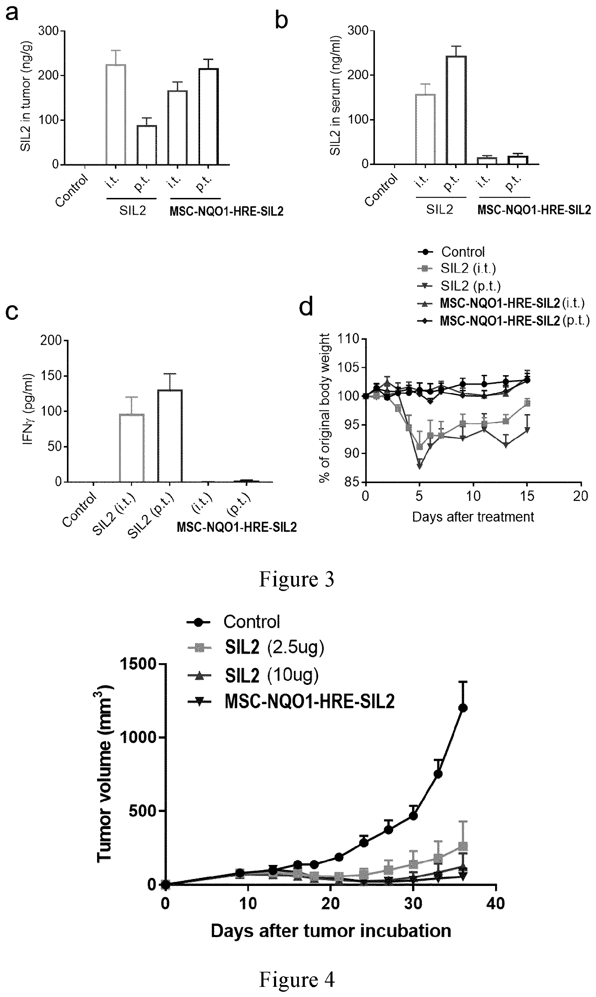

Figure 3 shows that NQO1/HRE-engineered MSCs expressing SIL2 (MSC-NQO1-HRE-SIL2) express SIL2 specifically in tumor tissues. (a) SIL2 levels in tumors after intratumoral (i.t.) or peritumoral (p.t.) injection of SIL2-Fc protein or of NQO1/HRE-engineered MSCs expressing SIL-2; (b) SIL2 levels in serum after intratumoral (i.t.) or peritumoral (p.t.) injection of SIL2-Fc protein or of NQO1/HRE-engineered MSCs expressing SIL-2; (c) IFN-γ levels in serum after intratumoral (i.t.) or peritumoral (p.t.) injection of SIL2-Fc protein or of NQO1/HRE-engineered MSCs expressing SIL-2; (d) changes in body weight of mice after intratumoral (i.t.) or peritumoral (p.t.) injection of SIL2-Fc protein or of NQO1/HRE-engineered MSCs expressing SIL-2; wherein the control is intratumoral injection of phosphate-buffered saline (PBS).
Figure 4 shows the effect of different doses of SIL2 protein and engineered MSCs expressing SIL2 (MSC-NQO1-BRE-SIL2) on the tumor volume. The control is injection of phosphate-buffered saline (PBS).

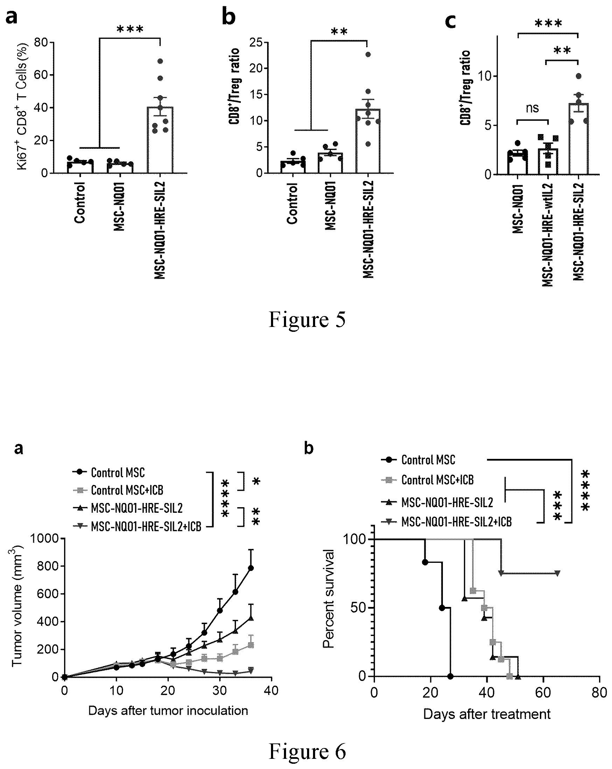

Figure 5 shows (a) the proportions of Ki67⁺CD8⁺ T cells, and (b and c) ratios of the number of CD8⁺ T cells to the number of Treg cells within tumors after injection of the control (PBS), engineered MSCs without tumor-suppressive cytokines (MSC-NQO1), engineered MSCs expressing wild-type IL-2 (wtIL2), and engineered MSCs expressing SIL2. Statistical significance: ns - not significant, * p<0.05, ** p<0.01, *** p<0.001.
Figure 6 shows the effects of engineered MSCs expressing SIL2 (MSC-NQO1-HRE-SIL2) and/or the effect of an immune checkpoint inhibitor on the tumor volume and survival of tumor-bearing mice. (a) Tumor volume and (b) survival time of the mice after injection of control MSCs (MSC-NQO1, without HRE or SIL2) (•), control MSC + immune checkpoint inhibitor (■)**,** MSC-NQO1-HRE-SIL2 (A), and MSC-NQO1-HRE-SIL2 + immune checkpoint inhibitor (▼). Statistical significance: * p<0.05, ** p<0.01, *** p<0.001, **** p<0.0001.

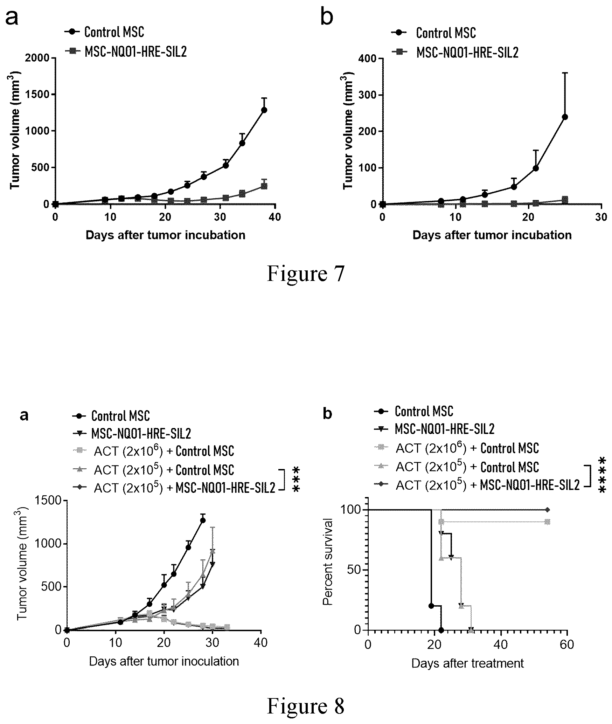

Figure 7 shows the effect of engineered MSCs expressing SIL2 (MSC-NQO1-HRE-SIL2) localized in one tumor on another distal tumor. (a) Changes in the volume of the right-side tumor and (b) changes in the volume of the left-side tumor in tumor-bearing mice after intratumoral injection of control MSCs (MSC-NQO1, without HRE or SIL2) or MSC-NQO1-HRE-SIL2 into the right-side tumor.
Figure 8 shows the effect of the combination of engineered MSCs expressing SIL2 (MSC-NQO1-HRE-SIL2) and adoptive cell therapy (ACT) on advanced tumors. (a) Changes in tumor volume and (b) survival percentage after treating the tumor with control MSC (MSC-NQO1), MSC-NQO1-HRE-SIL2, control MSC + two concentrations of ACT, and MSC-NQO1-HRE-SIL2 + low-concentration of ACT. Statistical significance: *** p<0.001, **** p<0.0001 (two-way ANOVA (a), log-rank test (b)).

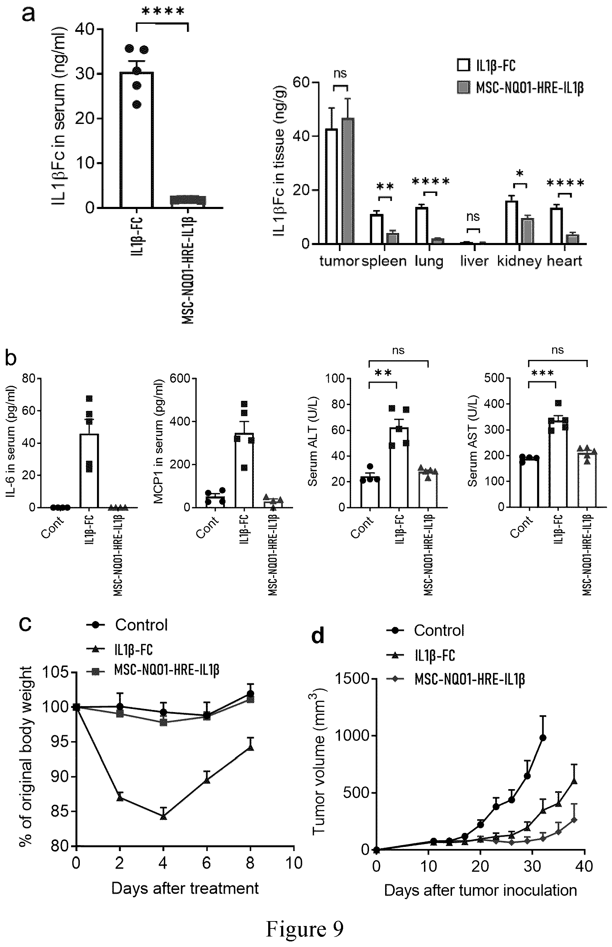

Figure 9 shows that engineered MSCs expressing IL1β (MSC-NQO1-HRE-IL1β) express IL1β specifically in tumor tissues. (a) IL1β levels in the tumor, various organs, and serum after intratumoral injection of IL1β protein or of MSC-NQO1-HRE-IL1β; (b) serum levels of inflammatory cytokines after intratumoral injection of a PBS control, IL1β protein, or MSC-NQO1-HRE-IL1β; (c) Changes in body weight of the mice after intratumoral injection of a PBS control, IL1β protein, or MSC-NQO1-HRE-IL1β; (d) Changes in tumor volume of the mice after intratumoral injection of a PBS control, IL1β protein, or MSC-NQO1-HRE-IL1β. Statistical significance: ns - not significant, * p<0.05, ** p<0.01, *** p<0.001, **** p<0.0001.

### DETAILED DESCRIPTION OF EMBODIMENTS

### Terminology and Definitions

In the present invention, the term "introduced NQO1 protein-coding sequence" refers to a nucleic acid sequence encoding an NQO1 protein that is introduced into the genome of a target cell (e.g. MSC) by a genetic modification method, thereby being distinguished from the native NQO1 gene in the genome of the cell. The NQO1 protein-coding sequence may be, for example, a coding sequence of human NQO1 protein (e.g., NCBI sequence NP_000894.1), a coding sequence of mouse NQO1 protein (e.g., SEQ ID No: 1), or a coding sequence of an NQO1 protein from other mammals, but is not limited thereto.

In the present invention, the term "introduced hypoxia response element (HRE)" refers to an HRE that is introduced into the genome of a target cell (e.g. MSC) by a genetic modification method, thereby being distinguished from the native HRE in the genome of the cell. Typically, downstream of the introduced HRE, a regulatory element and a target gene may be operably linked, to promote the specific expression of the target gene under a hypoxic condition. The sequence of the HRE may be derived from human or mouse, and may be from genes such as *Eno1, Epo,* VEGF-A, PGK, *Ldha,* ALDA, and GAPDH, for example, SEQ ID No: 2 derived from *Eno1.*

In the present invention, the "mesenchymal stem cells (MSCs)" used for engineering may be derived from mammals, such as humans, rats, or mice, for example, MSCs derived from their bone marrow, but not limited thereto.

In the present invention, the terms "sumIL-2" and "SIL-2" may be used interchangeably and refer to a mutant form of IL-2 that contains F42A, L80F, R81D, L85V, I86V, and I92F mutations on the basis of wild-type IL-2, which decrease the binding ability with CD25 and increase the binding affinity with IL2Rβγ receptor.

Herein, NQO1/HRE double-engineered MSCs expressing a target gene are denoted as "MSC-NQO1-HRE-(target gene)". For example, when the target gene is SIL-2, the NQO1/HRE-engineered MSCs expressing SIL-2 are denoted as "MSC-NQO1-HRE-SIL2". Similarly, for example, "MSC-NQO1" represents MSCs engineered with NQO1 only, without HRE or an HRE-regulated target gene; "MSC-NQO1-SIL2" represents MSCs engineered with NQO1 and comprising an introduced SIL-2 gene, but not engineered with HRE; and "MSC-SIL2" represents MSCs containing an introduced SIL2 gene but not engineered with NQO1 or HRE.

Various embodiments of the present invention are described below. It should be be understood by those skilled in the art that these embodiments do not limit the scope of the invention, but can be modified into equivalent embodiments that can be foreseen by those skilled in the art and can be combined with each other.

### I. NQO1-Engineered MSCs

The invention first provides an engineered mesenchymal stem cell (MSC) comprising in its genome an introduced NQO1 protein-coding sequence. NQO1 refers to NAD(P)H : quinone oxidoreductase 1. In order to improve the survival of adoptively transduced therapeutical MSCs within tumor tissues, NQO1 is made overexpress in MSCs. Surprisingly, as compared to non-engineered MSCs, the engineered MSCs overexpressing NQO1 of the present invention have a significantly increased survival in the tumor microenvironment (see Figure 1). This greatly increases the usefulness of MSCs as a cellular carrier within tumors, for example, for more persistent or stable delivery or production of anti-tumor molecules or other functional molecules within the tumor. The NQO1 protein-coding sequence in the engineered MSCs of the present invention may be a coding sequence of any NQO1 protein, such as human NQO1 or mouse NQO1 protein (e.g., SEQ ID No: 1), but not limited thereto.

The method for introducing the NQO1 protein-coding sequence into the MSC's genome may comprise: introducing a vector comprising the NQO1 protein-coding sequence (e.g. a lentiviral or adenoviral vector) into MSCs by transfection, transduction, infection, or other methods, or alternatively, gene-editing methods such as mRNA transfection or CRISPR/Cas9 systems can be used to insert the NQO1 protein-coding sequence into the MSC's genome, but the method is not limited thereto.

In one embodiment, the genome of the engineered MSC further comprises an introduced regulatory element and an introduced target gene, wherein the expression of the target gene is regulated by the regulatory element, and the target gene may be any gene. In one embodiment, the target gene encodes an immunostimulatory cytokine or an anti-tumor cytokine. In one embodiment, the target gene encodes any of the T-cell-stimulating cytokines IL-2, IL-1, IL-7, IL-21, IL-12, IL-15, and variants thereof. In one embodiment, the IL-1 is IL-1β. In one embodiment, the IL-2 variant is sumIL2 (SIL2). In one embodiment, when the engineered MSCs are administered to a tumor, the target gene may be expressed to produce anti-tumor factors to exert a specific anti-tumor function, and the engineered MSCs can enhance the anti-tumor effect of these anti-tumor factors due to their extended lifespan.

### II. HRE-Engineered MSCs

The invention further provides an engineered MSC comprising in its genome an introduced hypoxia response element (HRE), and optionally an introduced regulatory element and an introduced target gene located downstream of the HRE, wherein the expression of the target gene is regulated by the regulatory element. The inventors have found that, in the hypoxic environment within tumor tissues, the binding of HRE to HIF1α, which accumulates under hypoxia, enables activation of the downstream regulatory element (e.g. a promoter), thereby greatly promoting the expression of the gene downstream of the regulatory element (see Figure 2). This can significantly improve the efficiency and specificity of MSCs as a cellular carrier that delivers or produces anti-tumor molecules or other functional molecules within tumors. In one embodiment, the regulatory element includes a promoter. In one embodiment, the promoter is a minimal SV40 promoter. In one embodiment, the engineered MSC comprises HRE, the regulatory element, and the target gene that are operably linked.

The target gene may be any gene. In one embodiment, the target gene encodes an immunostimulatory cytokine or an anti-tumor cytokine. In one embodiment, the target gene encodes any of IL-2, IL-1, IL-7, IL-21, IL-12, IL-15, and variants thereof. In one embodiment, the IL-1 is IL-1β. In one embodiment, the IL-2 variant is sumIL2 (SIL2), which has a superior anti-tumor effect over wild-type IL-2 (see Figure 5).

The method for introducing the HRE, the regulatory element (e.g. a promoter), and the target gene into the MSC's genome may comprise introducing a vector comprising these nucleic acid sequences (e.g. a lentiviral or adenoviral vector) into MSC by transfection, transduction, infection, or other means. Alternatively, gene-editing methods such as mRNA transfection or CRISPR/Cas9 systems may be used to insert these nucleic acid sequences into the MSC's genome, but the method is not limited to the above methods. In one embodiment, the HRE and the regulatory element (e.g. a promoter) and the target gene downstream of the HRE are operably linked together and constructed in a lentiviral vector, and then the vector is used to transfect the engineered MSC.

### III. NQO1/HRE-Engineered MSCs

The invention further provides an engineered MSC comprising in its genome (i) an introduced NQO1 protein-coding sequence, and (ii) an introduced HRE, and optionally an introduced regulatory element and an introduced target gene downstream of the HRE, wherein the expression of the target gene is regulated by the regulatory element. In one embodiment, the regulatory element includes a promoter. In one embodiment, the promoter is a minimal SV40 promoter. In one embodiment, in the engineered MSC, the HRE, the regulatory element, and the target gene are operably linked. In one embodiment, the target gene encodes an immunostimulatory cytokine or an anti-tumor cytokine. In one embodiment, the target gene encodes any of IL-2, IL-1, IL-7, IL-21, IL-12, IL-15, and variants thereof. In one embodiment, the IL-1 is IL-1β. In one embodiment, the IL-2 variant is sumIL2.

The methods for introducing NQO1, HRE, the regulatory element (e.g. a promoter), and the target gene to be expressed into the MSC's genome are the same as those described above. In one embodiment, the NQO1 protein-coding sequence, the HRE, and the regulatory element (e.g. a promoter) and the target gene downstream of the HRE may be constructed together in a lentiviral vector, wherein the HRE, the regulatory element, and the target gene are operably linked, while the NQO1 coding sequence may not be operably linked to them but is regulated by an independent promoter; and then the MSC may be transduced with the lentiviral vector.

The NQO1/HRE-engineered MSC of the present invention can serve as a carrier to deliver immunostimulatory cytokines or anti-tumor cytokines (e.g. IL-1, IL-2) specifically to the tumor microenvironment, and showed a superior anti-tumor effect as compared to non-engineered MSCs expressing these cytokines or direct administration of the cytokines to the tumor (see Figures 2 and 4). Meanwhile, the immunostimulatory cytokines or anti-tumor cytokines expressed by the engineered MSCs are substantially confined within the tumor, avoiding the peripheral toxic side effects caused by systemic administration of these cytokines (see Figure 3). However, the anti-tumor effect of these MSCs was not limited to the local site of administration, but showed a further inhibitory effect to the growth of distal tumors (Figure 7). This is because the T cells activated and expanded by the engineered MSCs migrate to distal tumors and exert an anti-tumor effect. In addition, the inventors have also found that, the NQO1/HRE-engineered MSCs expressing IL-2 according to the present invention can improve the anti-tumor effect of immune checkpoint inhibitors (Figure 6), thereby being promising for treatment of advanced tumors resistant to immune checkpoint blockade (ICB) therapies. Moreover, they may further expand adoptive anti-tumor T cells and improve the efficacy of adoptive cells in treating cold tumors (Figure 8).

Nevertheless, the immunostimulatory cytokines or anti-tumor cytokines delivered or produced by the NQO1/HRE-engineered MSCs of the present invention as a cellular carrier are not limited to IL-2, but may be any cytokines with an immunostimulatory or anti-tumor effect, such as IL-1β. This is because NQO1 independently enhances the surviving ability of MSCs within the tumor microenvironment, and the promoting effect of HRE on the expression of the downstream regulatory element and target gene depends on the hypoxic environment in the tumor microenvironment. The application has confirmed that, when the target gene is IL-1β, it can also be expressed at a significant level and be relatively enriched within the tumor, causing low peripheral toxicity and showing a superior tumor suppression effect over separate injection of IL-1β protein (Figure 9).

### IV. Pharmaceutical Composition

The present invention further provides a pharmaceutical composition for treating cancer, comprising an engineered MSC that comprises in its genome (i) an introduced NQO1 protein-coding sequence, and/or (ii) an introduced hypoxia response element (HRE), and an introduced regulatory element and an introduced target gene downstream of the HRE, wherein the target gene encodes an immunostimulatory cytokine or an anti-tumor cytokine.

In one embodiment, the pharmaceutical composition further comprises an immune checkpoint inhibitor, such as anti-PD-L1 antibody, anti-PD-1 antibody, and anti-CTLA4 antibody. In one embodiment, the pharmaceutical composition further comprises adoptive T cells that specifically recognize tumor cells.

### V. Method for Treating Cancer

The present invention further provides a method for treating cancer, comprising administering a therapeutically effective amount of engineered MSCs to a subject in need thereof, wherein the MSC comprises in its genome (i) an introduced NQO1 protein-coding sequence, and/or (ii) an introduced hypoxia response element (HRE), and an introduced regulatory element and an introduced target gene downstream of the HRE, wherein the target gene encodes an immunostimulatory cytokine or an anti-tumor cytokine.

In one embodiment, the method further comprises administering an immune checkpoint inhibitor to the subject, such as an anti-PD-L1 antibody, an anti-PD-1 antibody, and an anti-CTLA4 antibody. In one embodiment, the method further comprises administering adoptive cell therapy (ACT) to the subject, such as CAR-T cell therapy, TIL therapy, and TCR-T therapy. In one embodiment, the subject is a human.

### VI. Use in Manufacture of Medicament

The present invention further provides use of engineered MSCs in the manufacture of a medicament for treating cancer, wherein the MSC comprises in its genome (i) an introduced NQO1 protein-coding sequence, and/or (ii) an introduced hypoxia response element (HRE), and an introduced regulatory element and an introduced target gene downstream of the HRE, wherein the target gene encodes an immunostimulatory cytokine or an anti-tumor cytokine.

In one embodiment, the medicament further comprises an immune checkpoint inhibitor, such as an anti-PD-L1 antibody, an anti-PD-1 antibody, and an anti-CTLA4 antibody. In one embodiment, the medicament further comprises adoptive T cells that specifically recognize tumor cells.

In the embodiments of the pharmaceutical composition, of the method for treating cancer, and of the use in manufacture of a medicament described above, the regulatory element may include a promoter. In one embodiment, the promoter is a minimal SV40 promoter. In one embodiment, in the engineered MSC, the HRE, the regulatory element, and the target gene are operably linked. In one embodiment, the target gene encodes any of IL-2, IL-1, IL-7, IL-21, IL-12, IL-15, and variants thereof. In one embodiment, the IL-1 is IL-1β. In one embodiment, the IL-2 variant is sumIL2. In one embodiment, the cancer may be selected from B-cell lymphoma, bronchial cancer, prostate cancer, bladder cancer, brain or central nervous system cancer, peripheral nervous system cancer, esophageal cancer, cervical cancer, uterine or endometrial cancer, oral cancer, laryngeal cancer, salivary gland cancer, thymus cancer, adrenal cancer, osteosarcoma, chondrosarcoma, liposarcoma, testicular cancer, malignant fibrous histiocytoma, colorectal cancer, melanoma, gastric cancer, pancreatic cancer, lung cancer, liver cancer, renal cancer, cholangiocarcinoma, small intestine cancer or appendiceal cancer, squamous cell carcinoma, breast cancer, and ovarian cancer.

### Examples

Hereinafter, the embodiments of the present invention will be further described with reference to Examples. However, it should be understood that the Examples are provided only for specific illustration and do not limit the scope of the invention in any way.

### Materials and Methods

### 1. Mice

C57BL/6J, BALB/c, and C57BL/6J-Tg (TcraTcrb) 1100Mjb/J (OT1 TCR transgenic) mice, aged 6-8 weeks, were purchased from the Jackson Laboratory. Mice were housed in an SPF environment, and all animal experiments complied with the implementing regulations for laboratory animal management of Tsinghua University.

### 2. Cell Lines and Reagents

The MC38 (mouse colon cancer cells), CT26 (mouse colon cancer cells), and B16F10 (mouse cutaneous melanoma cells) cell lines were purchased from the American Type Culture Collection (ATCC). The MC38-OVA and B6-OVA cell lines were obtained by transfecting MC38 or B16F10 with lentiviruses expressing the chicken OVA protein gene. The culture medium for cell lines was DMEM (containing 10% heat-inactivated fetal bovine serum (FBS), 100 U/ml penicillin, and 100 µg/ml streptomycin), and the culture conditions were 37°C with 5% CO₂. Anti-PD-L1 antibody (Atezolizumab) and anti-CTLA-4 antibody (9D9) were purchased from BioXCell.

### 3. Isolation, Culture, and Viral Transduction of Bone Marrow-Derived MSCs

Bone marrow cells were isolated from 6-8-week-old female C57BL/6J or BALB/c mice, resuspended in Mesencult Expansion Media (Stemcell Technologies) containing 1 µM GW2580, seeded in 10 cm cell culture dishes, and cultured under a hypoxic condition (1% oxygen concentration). After 24 hours, cells that were not adhered to the bottom of the culture dishes were removed by changing the medium. Fresh medium was replaced every 3 days, and the cells were passaged at a 1:3 ratio when the cell confluence reached 60%. MSCs were identified by flow cytometry detecting the expression of positive and negative surface markers.

Live MSCs were transfected with lentiviruses expressing the EGFP gene or the EGFP + mouse NQO1 protein-coding gene (SEQ ID No: 1), and EGFP-positive cells were sorted by flow cytometry to obtain MSC-EGFP or MSC-EGFP-NQO1 cells.

The hypoxia response element (HRE) (SEQ ID No: 2) and minimal SV40 promoter (SEQ ID No: 3) were obtained from the HIF-1 reporter p2.1 plasmid by PCR. After the cloned sumIL2 coding sequence was linked to the HRE-SV40 sequence, it was inserted into a lentiviral vector to be packaged into lentiviruses, and the MSCs were then transfected with the lentiviruses and sorted to obtain MSC-NQO1-HRE-SIL2 or MSC-HRE-SIL2. SIL2 expression was detected by ELISA. Lentiviral vectors containing HREs from genes such as Epo, VEGF-A, PGK, Ldha, ALDA, or GAPDH can be obtained by a similar method.

### 4. Establishment and treatment of Mouse Tumor Model

MC38 (1×10⁶), CT26 (5×10⁵), MC38-OVA (1×10⁶), and B16-OVA (3×10⁵) cells were subcutaneously inoculated into the right dorsum of mice to develop tumors, and then the mice were randomly grouped. The mice were injected with 20 µg of SIL2 protein, or 1×10⁶ control MSCs (MSC-NQO1), or 1×10⁶ MSC-NQO1-HRE-SIL2 at specific time points. With respect to the therapy combined with immune checkpoint blockade against advanced tumors, from day 14 after MC38 tumor inoculation (the tumors were approximately 150 mm³), the C57BL/6J mice were intraperitoneally injected with 100 µg of anti-PD-L1 + 100 µg of anti-CTLA4 combined with peritumoral injection of 1×10⁶ control EMSCs or MSC-NQO1-HRE-SIL2 cells. The injections were performed every 3 days, for a total of two times. With respect to the therapy combined with adoptive cell therapy, the C57BL/6J mice were subcutaneously injected with 3×10⁵ B16-OVA, and OT-1 T cells (1×10⁶ or 2×10⁵) pre-activated with the OT-1 peptide were intravenously injected on day 11, and 1×10⁶ control EMSCs or MSC-NQO1-HRE-SIL2 cells were peritumorally injected on day 11 and day 14. The tumor length (a), width (b), and height (c) were measured twice a week, and the tumor volume was calculated as a*b*c/2. Survival curves of the tumor-bearing mice were recorded. Mice were euthanized when the length, width, or height of the tumor exceeded 2 cm, or the size of the tumor exceeded 1,500 mm³, or the body weight of the tumor-bearing mice decreased by more than 20%.

### 5. Tumor Tissue Digestion

Tumor tissue was resuspended in a digestion buffer (RPMI medium containing 2% FBS, 1 mg/ml collagen IV, and 100 µg/ml DNase I) and incubated at 37°C, 120 rpm to be digested for 45 minutes. The tumor suspension was passed through a 70 µm cell strainer to remove large debris or undigested tumor tissue blocks. The filtrate was washed twice with PBS containing 2 mM EDTA and then resuspended in a FACS buffer.

### 6. Flow Cytometry

Anti-FcγIII/II receptor (clone 2.4G2) antibody was added to the tumor cell suspension, which was then incubated on ice for 15 minutes to block non-specific binding, followed by addition of corresponding fluorochrome-conjugated antibodies and staining at 4°C in dark for 30 minutes. Fixable viability Dye eFluor^{™} 506 or eFluor^{™} 780 staining were used to exclude dead cells. Foxp3 intranuclear staining was performed using the True-Nuclear^{™} transcription factor buffer set (BioLegend) according to the instructions. Data were acquired by CytoFLEX flow cytometer (Beckman Coulter) and analyzed with FlowJo software (Tree Star).

### 7. Preparation of Tumor Tissue Homogenate

On day 9, CT26 tumor-bearing mice were intratumorally injected with 20 µg of sumIL2 protein or 1×10⁶ EMSC-sumIL2. Five days later, tumor tissues were collected, minced into small pieces, resuspended with a Cell Lysis Kit (Bio-Rad Laboratories), and prepared into a homogenate by a FastPrep-24 5G homogenizer, followed by centrifugation at 13,000 rpm for 20 minutes. The supernatant was collected and stored at -80°C.

### 8. Cytometric Bead Array (CBA) and ELISA Analysis of Serum and Tissue Samples

The CBA Mouse Th1/Th2/Th17 Kit (BD Biosciences) was used to measure the cytokine level in the serum and tumor tissue homogenate of the mice according to the instructions. For ELISA, a 96-well microplate (Corning Costar) was used, and 2 µg/mL (100 µL/well) of capture antibody was added to the plate for adsorption overnight at 4°C. The plate was washed with PBS, and blocked with a blocking buffer (PBS containing 0.05% TWEEN-20 and 5% skim milk), and then the serum or tumor homogenate diluted with the blocking buffer were added and incubated at 37°C for 1.5 hours. After washing the plate with PBST, alkaline phosphatase-labelled goat anti-human IgG secondary antibody was added and incubated at 37°C for 50 minutes. After washing with PBST, 100 µL of p-nitrophenyl phosphate was added for color development, and then the plate was read at 405 nm using SPECTROstar Nano (BMG LABTECH).

### 9. Preparation of SumIL2-Fc Protein

For plasmid construction, the IL-2 mutant SumIL-2 (abbreviated as SIL2, containing F42A, L80F, R81D, L85V, I86V, and I92F mutations) was connected to the N-terminus of the Fc derived from hIgG1 (containing L233A, L234A, and P329G mutations) (coding sequence: SEQ ID No: 4). FreeStyle^{™} 293-F cells were transiently transfected with the plasmid. On day 6 after transfection, the supernatant was collected and purified for SIL2-Fc protein using a Protein A affinity chromatography column.

### 10. Preparation of IL-1β-Fc Protein

IL-1β-Fc protein (coding sequence: SEQ ID No: 5) was obtained by a method similar to that for the preparation of SumIL2-Fc protein.

### 11. Data Analysis

Before treatment, tumor-bearing mice were randomly divided into different groups based on the tumor volume. Data were analyzed using GraphPad Prism statistical software and presented as mean ± SEM. Tumor growth curves were analyzed using two-way ANOVA to calculate p-values, mouse survival curves were analyzed using a log-rank test to calculate p-values, and other data were analyzed using unpaired two-tailed t-tests. p < 0.05 indicated a significant difference.

### 12. Sequence Description

SEQ ID No: 1 The nucleic acid sequence encoding mouse NQO1 protein
SEQ ID No: 2 The sequence of the hypoxia response element (HRE) derived from *Eno1* gene
SEQ ID No: 3 The sequence of minimal SV40 promoter
SEQ ID No: 4 The nucleic acid sequence encoding sumIL2-Fc
SEQ ID No: 5 The nucleic acid sequence encoding IL1β-Fc
SEQ ID No: 6 The sequence of the hypoxia response element (HRE) derived from EPO gene
SEQ ID No: 7 The sequence of the hypoxia response element (HRE) derived from VEGF-A gene
SEQ ID No: 8 The sequence of the hypoxia response element (HRE) derived from PGK1 gene
SEQ ID No: 9 The sequence of the hypoxia response element (HRE) derived from LdhA gene
SEQ ID No: 10 The sequence of the hypoxia response element (HRE) derived from ALDA gene
SEQ ID No: 11 The sequence of the hypoxia response element (HRE) derived from GAPDH gene

### Example 1. NQO1-Engineered MSCs Show Longer Survival Time in Tumor

To enhance the survival of MSCs in tumor tissues inside the body, MSCs were transfected with a lentiviral vector carrying EGFP and NQO1 gene sequences as described above to obtain NQO1-engineered MSCs ("MSC-EGFP-NQO1"), while MSCs carrying only EGFP were used as a control ("MSC-EGFP"). To evaluate the survival time of NQO1-engineered MSCs in tumor tissues inside the body, 8-week-old female C57BL/6J mice (n=5) were subcutaneously inoculated with 1×10⁶ MC38 cells. On day 9, 5×10⁵ MSC-EGFP or MSC-EGFP-NQO1 cells were injected intratumorally. Five days after the injection, tumor tissues were collected, and the number of CD45⁻GFP⁺ MSCs in the tumor was analyzed by flow cytometry. As shown in Figures 1a and 1b, MSC-EGFP-NQO1 cells were significantly more than MSC-EGFP cells in the tumor, indicating that MSCs expressing NQO1 were better adapted to the tumor microenvironment and had a longer survival time.

### Example 2. HRE-Engineered MSC-NQO1 Expresses Anti-Tumor Factors Specifically in Hypoxic Tumor Tissue

To enable MSC-NQO1 to express anti-tumor factors specifically in tumor tissues and avoid peripheral toxicity, a hypoxia-inducible promoter element was used to regulate the gene expression of the target anti-tumor factor IL-1β (IL-1β-Fc) or SIL-2 (sumIL-2-Fc). The hypoxia-inducible promoter element contained an *Eno1*-derived hypoxia response element (HRE) obtained by PCR from the HIF-1 reporter P2.1 plasmid, and a minimal SV40 promoter. MSC-NQO1 cells were transfected with a lentiviral vector carrying the target gene (IL-1β or SIL-2) under the regulation of the HRE-SV40 promoter or with a lentiviral vector lacking the HRE element but carrying the target gene, respectively, and the cells were then cultured under a normoxic (20%) or hypoxic (1%) condition for 24 hours. The concentration of the expressed anti-tumor factor IL-1β or SIL-2 in the supernatant was measured by ELISA (n=4). MSC-NQO1-HRE-IL-1β or -SIL-2 (gray bars in Figure 2b and 2c) expressed approximately 10 times more IL-1β or SIL-2 under the hypoxic condition than under the normoxic condition. In contrast, the control MSC-NQO1-IL-1β or -SIL-2 (black bars in Figure 2b and 2c) showed no significant difference in the production of IL-1β or SIL-2 between hypoxic and normoxic conditions, both far lower than the level secreted by MSC-NQO1-HRE-IL-1β or -SIL-2 under the hypoxic condition. This indicates that HRE-engineered MSCs can promote the expression of a target gene specifically under a hypoxic condition.

To evaluate the *in vivo* anti-tumor effect of the engineered MSCs, 8-week-old female C57BL/6J mice were subcutaneously inoculated with 1×10⁶ MC38 cells. On days 9 and 12, 1×10⁶ engineered MSC-NQO1-SIL-2, engineered MSC-NQO1-BRE-SIL-2, or MSC-SIL-2 (i.e. MSCs carrying only SIL-2 gene without NQO1 or HRE element) were injected intratumorally, and the tumor volume was measured on a daily basis (n=5 or 6). As shown in Figure 2d, MSC-NQO1-SIL-2 showed an improved anti-tumor effect as compared to MSC-SIL-2, indicating that the extended survival of NQO1-engineered MSCs improved their anti-tumor capability. MSC-NQO1-HRE-SIL-2 demonstrated the strongest anti-tumor effect, indicating that the additional introduction of HRE further enhanced its anti-tumor capability.

### Example 3. Engineered MSC-NQO1-HRE-SIL-2 Expresses sumIL-2 Specifically in Tumor Tissue and Avoids Peripheral Toxicity

A mutant form of IL-2, sumIL-2 (SIL-2), was constructed as described above. As compared to wild-type IL-2, sumIL-2 shows weakened binding to IL2Rα and enhanced binding to IL2Rβ, and thus tends to activate effector CD8⁺ T cells and reduce activation of suppressive Treg cells. To extend its half-life, sumIL-2 was fused to the Fc portion of hIgG, wherein L233A, L234A, and P329G mutations had been introduced into the Fc to eliminate cell-dependent cytotoxicity (ADCC) and complement-dependent cytotoxicity (CDC), thereby avoiding the deletion effect on activated T cells. While sumIL-2-Fc had a better anti-tumor effect than IL-2-Fc, its toxicity was also increased accordingly.

In this Example, a lentiviral vector carrying NQO1-HRE-sumIL2-Fc was constructed (wherein the expression of sumIL-2-Fc was regulated by HRE, but NQO1 was regulated by an element independent of HRE), packaged into virus particles, and transfected into MSC cells to obtain "MSC-NQO1-HRE-SIL-2." To evaluate the ability of MSCs as a carrier to deliver sumIL-2 to tumor tissues, 8-week-old female BALB/c mice were subcutaneously inoculated with 5×10⁵ CT26 tumor cells. On day 9, 20 µg of SIL-2-Fc protein or 1×10⁶ MSC-NQO1-HRE-SIL-2 cells were injected intratumorally or peritumorally. Five days after the injection, tumor tissues and serum were collected, and the level of sumIL-2 in tumor tissue homogenates and the serum were measured by ELISA (n=5 or 6).

The results showed that the concentration of sumIL-2 in tumor tissues after intratumoral injection of SIL-2-Fc protein or MSC-NQO1-HRE-SIL-2 cells was similar to each other, and was higher than that in tumors tissues after peritumoral injection of SIL-2-Fc protein (Figure 3a). Interestingly, the concentration of sumIL-2 protein produced after peritumoral injection of MSC-NQO1-HRE-SIL-2 cells was similar to that of intratumoral injection, indicating that peritumorally injected MSCs were able to home to the tumor tissue and secrete sumIL-2 protein. Intratumoral or peritumoral injection of sumIL-2 protein resulted in a high concentration of sumIL-2 detected in the serum. In contrast, both intratumoral and peritumoral injections of MSC-NQO1-HRE-SIL-2 cells led to only a tiny amount of sIL-2 protein detected in the serum (Figure 3b). It is known that a high concentration of sumIL-2 protein in the serum activates peripheral T and NK cells, producing cytokines to cause peripheral toxicity. Accordingly, the mice injected with sumIL-2 protein intratumorally or peritumorally showed a significantly elevated IFN-γ level in the serum as detected by CBA. In contrast, the mice injected with MSC-NQO1-HRE-SIL-2 cells intratumorally or peritumorally showed no detectable IFN-γ protein in the serum (Figure 3c). After intratumoral or peritumoral injection of sumIL-2 protein, the mice showed a significant decrease in body weight; in contrast, the mice injected with MSC-NQO1-HRE-SIL2 cells intratumorally or peritumorally showed no significant changes in body weight as compared to the untreated control group.

The results demonstrated that intratumoral or peritumoral injection of MSC-NQO1-HRE-SIL2 cells can secrete a high level of sumIL2 protein specifically within tumor tissues, while producing substantially no SIL2 protein and its associated toxicity in peripheral tissues.

### Example 4. Significant Tumor Suppression Effect of MSC-NQO1-HRE-SIL2

The anti-tumor effect of MSC-NQO1-HRE-SIL2 was evaluated using a CT26 colorectal cancer mouse tumor model. 8-week-old Female BALB/c mice were subcutaneously inoculated with 5×10⁵ CT26 tumor cells. On days 9 and 12 after inoculation, SIL2-Fc protein (sumIL-2-Fc) was injected intratumorally at the doses shown in Figure 4, or 1×10⁶ MSC-NQO1-HRE-SIL2 cells were injected peritumorally. The mice were observed twice a week, and tumor sizes were measured (n=5). The results are shown in Figure 4. At doses that did not cause peripheral toxicity, 1×10⁶ MSC-NQO1-HRE-SIL2 cells were able to significantly inhibit tumor growth, with a slightly better treatment effect than 2.5 µg or 10 µg of SIL2-Fc protein.

### Example 5. Selective Expansion of CD8+ T Cells in Tumor by Administration of MSC-NQO1-HRE-SIL2

To further analyze the regulation of anti-tumor immunity by administration of MSC-NQO1-HRE-SIL2, 8-week-old female C57BL/6J mice were subcutaneously inoculated with 5×10⁵ CT26 tumor cells. On days 9 and 12 after inoculation, 1×10⁶ MSC-NQO1 cells, PBS control, or MSC-NQO1-HRE-SIL2 cells were injected peritumorally. Five days after the second injection, tumor tissues were isolated, and digested into single-cell suspensions with a digestion solution (containing 1 mg/ml collagenase IV and 100 µg/ml DNase I), and the number of different cell subsets was counted by flow cytometry. The results are shown in Figures 5a and 5b. MSC-NQO1-HRE-SIL2 significantly expanded CD8⁺ T cells, and the ratio of CD8⁺T/Treg cells was significantly higher than that in other groups.

To further analyze whether SIL2, as compared to wtIL2, was able to expand CD8⁺ T cells more specifically rather than Treg cells, 8-week-old female BALB/c mice were subcutaneously inoculated with 5×10⁵ CT26 tumor cells. On days 9 and 12 after inoculation, 1×10⁶ MSC-NQO1 cells, MSC-NQO1-HRE-SIL2 cells, or MSC-NQO1-HRE-wtIL2 cells (expressing wild-type IL-2) were injected intratumorally. Five days after the second injection, tumor tissues were isolated and digested into single-cell suspensions with a digestion solution (containing 1 mg/ml collagenase IV and 100 µg/ml DNase I), and the number of different cell subsets was counted by flow cytometry. The results are shown in Figure 5c. The ratio of CD8⁺T/Treg cells in the MSC-NQO1-HRE-SIL2 treatment group was significantly higher than that in other groups. In contrast, wtIL2 not only expanded anti-tumor CD8+ T cells within the tumor but also significantly expanded immunosuppressive Treg cells, and thus CD8⁺ T/Treg ratio in the MSC-NQO1-HRE-wtIL2 group was not significantly different from that in the MSC-NQO1 group. These results indicate that MSC-NQO1-HRE-SIL2 can selectively expand CD8⁺ T cells in tumors, thereby inhibiting tumor growth.

### Example 6. MSC-NQO1-HRE-SIL2 Improved Anti-tumor Effect of Immune Checkpoint Inhibitor

Immune checkpoint inhibitors have demonstrated good anti-tumor effects in clinical settings, but only a portion of patients can respond to the immune checkpoint blockade (ICB) therapy, and some patients who were responsive in an early stage became non-responsive in a later stage. To evaluate whether MSC-NQO1-HRE-SIL2 can improve the efficacy of the immune checkpoint inhibitor therapy, 8-week-old female C57BL/6J mice were subcutaneously inoculated with 1×10⁶ MC38 cells, and late-stage tumors were selected for treatment. On days 14 and 17 of tumor growth, 100 µg of anti-PD-L1 + anti-CTLA4 antibodies were injected intraperitoneally, or 1×10⁶ MSC-NQO1-HRE-SIL2 cells were injected peritumorally. Tumor growth or survival curves of the mice were recorded (n=5).

The results are shown in Figure 6. The MSC-NQO1-HRE-SIL2 monotherapy group (▲) partially inhibited the growth of late-stage tumors but did not eliminate the tumors. The immune checkpoint inhibitor plus control MSC group (■) showed a slightly better effect but still only partially inhibited tumor growth. In contrast, the combination therapy group with immune checkpoint inhibitors plus MSC-NQO1-HRE-SIL2 (▼) desirably significantly inhibited tumor growth (Figure 6a). According to the survival curves, the MSC-NQO1-HRE-SIL2 monotherapy group (▲) or the immune checkpoint inhibitor plus control MSC group (■) extended the survival of tumor-bearing mice up to approximately 50 days, while the combination therapy group of immune checkpoint inhibitors plus MSC-NQO1-HRE-SIL2 (▼) eliminated 75% of the tumors, and extended the survival time of the mice to 70 days or longer.

The results above indicate that MSC-NQO1-HRE-SIL2 not only inhibits tumor growth on its own but also can improve the anti-tumor effect of an immune checkpoint inhibitor, and showed efficacy even on late-stage tumors resistant to the immune checkpoint blockade (ICB) therapy.

### Example 7. Locally Produced sumIL2 by MSC-NQO1-HRE-SIL2 in Tumor Activates T Cells and Effectively Inhibits Growth of Distal Tumor

A subcutaneously inoculated dual-tumor mouse model was used to evaluate whether local administration of MSC-NQO1-HRE-SIL2 could induce a systemic anti-tumor immune response and control distal tumors away from the primary tumor. 8-week-old Female C57BL/6J mice were subcutaneously inoculated with 1×10⁶ MC38 tumor cells on the right side on day 0, and with 5×10⁵ MC38 tumor cells on the left side on day 2. On days 9 and 12, 1×10⁶ MSC-NQO1-HRE-SIL2 cells or control MSCs were injected intratumorally into the right-side tumor. Tumor sizes on both the left and right sides were recorded simultaneously twice a week (n=7-10).

The results are shown in Figure 7. As compared to the control MSCs, MSC-NQO1-HRE-SIL2 not only significantly inhibited the growth of the treated tumor on the right side, but also significantly inhibited the growth of the distal tumor on the left side. This indicates that MSC-NQO1-BRE-SEL2 can activate anti-tumor immune responses locally within a tumor and further inhibit the growth of a distal tumor.

### Example 8. Administration of MSC-NQO1-HRE-SIL2 Improved Anti-tumor Effect of Adoptive Cell Therapy (ACT) on Late-Stage Tumors

Since SIL2 can effectively expand CD8⁺ T cells, it is contemplated that it can be combined with the adoptive T cell therapy to treat "cold" tumors with a low degree of immune cell infiltration. B16 melanoma was selected for a cold tumor model. 8-week-old Female C57BL/6J mice were subcutaneously inoculated with 3×10⁵ B16-OVA tumor cells on day 0. On day 9, OT-1 T cells pre-activated with the OT-1 peptide were injected intravenously at the amounts shown in Figure 8, and 1×10⁶ control MSCs (MSC-NQO1) or MSC-NQO1-HRE-SIL2 cells were injected peritumorally to be combined with the ACT as shown in the figure. Tumor sizes and survival of the mice were recorded twice a week (n=5-10).

The results are shown in Figures 8a and 8b. MSC-NQO1-HRE-SIL2 (▼) or the ACT (2×10⁵) + control MSC (▲) only partially inhibited the growth of B16-OVA tumor cells, and the tumor-bearing mice eventually died. In contrast, the combination therapy group of MSC-NQO1-HRE-SIL2 and ACT (2×10⁵) (◆) significantly inhibited and even eliminated the tumors and extended mouse survival, with a treatment effect comparable to that of a 10-fold higher ACT dose (2×10⁶) (■). These results indicate that MSC-NQO1-HRE-SIL2 can expand adoptive anti-tumor T cells and improve the efficacy of adoptive cell therapy against cold tumors.

### Example 9. Engineered MSC-NQO1-HRE-IL1βExpresses IL1β Specifically in Tumor Tissue, Avoiding Peripheral Toxicity

A lentiviral vector carrying NQO1-HRE-IL1β-Fc (the expression of IL1β-Fc is regulated by the HRE element) was constructed in a similar way as described in Example 3, packaged into virus and used to transfect MSCs, and the transfected cells were named "MSC-NQO1-HRE-IL1B." The expression level of IL1β in tumor tissues and the anti-tumor effect of these MSCs were tested in a similar way as described in Example 3.

The results are shown in Figure 9a. After intratumoral injection of IL1β protein or MSC-NQO1-HRE-IL1β cells, MSC-NQO1-HRE-IL1β expressed IL1β within the tumor, while the expression levels in the serum and in other organs were significantly lower than in the tumor, and also lower than the case of direct injection of IL1β protein. Additionally, after injection of MSC-NQO1-HRE-IL1β, inflammatory cytokines IL-6 and MCP1, as well as liver damage indicators ALT and AST in the serum showed no significant difference from those in the control group (here, the control was the group injected intratumorally with PBS), while these inflammatory factors were significantly increased after injection of IL1β protein (Figure 9b). This indicates that administration of IL1β protein can cause peripheral tissue toxicity, while expression of MSC-NQ1O-HRE-IL1β specifically within the tumor does not cause peripheral tissue toxicity.

Moreover, in terms of the tumor suppression effect, MSC-NQO1-HRE-IL1β were significantly superior to IL1β protein with respect to tumor volume and body weight of tumor-bearing mice (Figures 9b and c).

The technical concepts and specific embodiments of the present invention have been described hereinabove. However, it should be understood that the specific embodiments described above do not limit the scope of the invention in any way. It will be understood by those skilled in the art that various modifications and/or variations can be made to the invention as shown in the specific embodiments without departing from the essence of the invention. The modified and/or varied embodiments also fall within the scope of the invention. Therefore, the embodiments of the present invention are illustrative and not restrictive.

## Claims

1. An engineered mesenchymal stem cell (MSC) comprising in its genome an introduced NQO1 protein-coding sequence.

2. The engineered mesenchymal stem cell of claim 1, further comprising in its genome an introduced regulatory element and an introduced target gene, wherein the expression of the target gene is regulated by the regulatory element.

3. An engineered mesenchymal stem cell (MSC) comprising in its genome an introduced hypoxia response element (HRE).

4. An engineered mesenchymal stem cell (MSC) comprising in its genome an introduced NQO1 protein-coding sequence and an introduced hypoxia response element (HRE).

5. The engineered mesenchymal stem cell of claim 3 or 4, further comprising in its genome an introduced regulatory element and an introduced target gene downstream of the HRE, wherein the expression of the target gene is regulated by the regulatory element.

6. The engineered mesenchymal stem cell of claim 5, wherein the HRE and the regulatory element and the target gene downstream of the HRE are operably linked.

7. The engineered mesenchymal stem cell of claim 2 or 5, wherein the regulatory element comprises a promoter.

8. The engineered mesenchymal stem cell of claim 2 or 5, wherein the target gene encodes an anti-tumor protein, preferably an immunostimulatory cytokine or an anti-tumor cytokine.

9. The engineered mesenchymal stem cell of claim 2 or 5, wherein the target gene encodes any of IL-2, IL-1, IL-7, IL-21, IL-12, IL-15, and variants thereof, preferably sumIL-2 or IL-1β.

10. The engineered mesenchymal stem cell of any of claims 1 to 5, wherein the introduction is performed through lentiviral transduction, adenoviral transduction, or mRNA transfection.

11. The engineered mesenchymal stem cell of claim 1 or 4, wherein the NQO1 protein-coding sequence is SEQ ID NO: 1.

12. The engineered mesenchymal stem cell of claim 3 or 4, wherein the sequence of the HRE is derived from any of *Eno1, Epo,* VEGF-A, PGK, *Ldha,* ALDA, and GAPDH genes.

13. The engineered mesenchymal stem cell of claim 3 or 4, wherein the sequence of the HRE is selected from SEQ ID NO: 2 and SEQ ID NOs: 6-11, preferably SEQ ID NO: 2.

14. A pharmaceutical composition for treating cancer, comprising the engineered mesenchymal stem cell of claim 8 or 9.

15. The pharmaceutical composition of claim 14, further comprising a second anti-cancer agent, such as an immune checkpoint inhibitor and/or adoptive T cells that recognize cancer cells.

16. Use of the engineered mesenchymal stem cell of claim 8 or 9 in the manufacture of a medicament for treating cancer.

17. The pharmaceutical composition of claim 14 or the use of claim 16, wherein the cancer is selected from B-cell lymphoma, bronchial cancer, prostate cancer, bladder cancer, brain or central nervous system cancer, peripheral nervous system cancer, esophageal cancer, cervical cancer, uterine or endometrial cancer, oral cancer, laryngeal cancer, salivary gland cancer, thymus cancer, adrenal cancer, osteosarcoma, chondrosarcoma, liposarcoma, testicular cancer, malignant fibrous histiocytoma, colorectal cancer, melanoma, gastric cancer, pancreatic cancer, lung cancer, liver cancer, renal cancer, cholangiocarcinoma, small intestine cancer or appendiceal cancer, squamous cell carcinoma, breast cancer, and ovarian cancer.

18. Use of NQO1 protein for extending the survival time of mesenchymal stem cells within tumor tissues.

19. Use of a hypoxia response element (HRE) for enhancing the expression of a target gene in mesenchymal stem cells under a hypoxic condition.

20. The use of claim 19, wherein the hypoxic condition is tumor microenvironment.

21. The use of claim 19, wherein the genome of the mesenchymal stem cell comprises the introduced HRE as well as an introduced regulatory element and an introduced target gene downstream of the HRE, and the expression of the target gene is regulated by the regulatory element.

22. Use of a combination of an NQO1 protein-coding sequence and a hypoxia response element (HRE) in the manufacture of a pharmaceutical composition which comprises mesenchymal stem cells carrying a target gene.

23. The use of claim 22, wherein the genome of the mesenchymal stem cell comprises the introduced NQO1 protein-coding sequence, the introduced HRE, and an introduced regulatory element and an introduced target gene downstream of the HRE, and the expression of the target gene is regulated by the regulatory element.

24. Use of the engineered mesenchymal stem cell of any of claims 1 to 13 as a carrier for delivering a target gene.

25. The use of claim 24, wherein the carrier is used for delivering the target gene to tumor tissue, and the target gene is a tumor therapeutic gene, preferably a gene encoding an anti-tumor protein, more preferably a gene encoding an immunostimulatory cytokine or an anti-tumor cytokine.
